(19) Europäisches Patentamt
European Patent Office
Office européen des brevets

(11) Publication number: **0 017 376**
**A1**

# (12) EUROPEAN PATENT APPLICATION

(21) Application number: 80300817.6

(22) Date of filing: 18.03.80

(51) Int. Cl.³: C 07 C 103/82
C 07 D 307/14, A 61 K 31/165
A 61 K 31/34

(30) Priority: 30.03.79 JP 37119/79
02.04.79 JP 39921/79
04.04.79 JP 40735/79

(43) Date of publication of application:
15.10.80 Bulletin 80/21

(84) Designated Contracting States:
AT BE CH DE FR GB IT LU NL SE

(71) Applicant: KISSEI PHARMACEUTICAL CO. LTD
No. 105 Nomizo, Yoshikawa-ku
Matsumoto-shi, Nagano(JP)

(72) Inventor: Kamijo, Tetsuhide
2525, Oaza Hirookayoshida
Shiojiri-shi Nagano(JP)

(72) Inventor: Yamamoto, Ryoji
No. 11-31 Nonizonishi 1-chome
Matsumoto-shi Nagano(JP)

(72) Inventor: Yamaura, Masanori
No. 6-3-5 Soya, Ichikawa-shi
Chiba(JP)

(72) Inventor: Ajisawa, Yukiyoshi
No. 10-4 Minato Okaya-shi
Nagano(JP)

(74) Representative: Roberts, Peter William et al,
Marks & Clerk Alpha Tower ATV Centre
Birmingham B1 1TT(GB)

## (54) Phenylacetamide compounds and an analgesic composition containing them.

(57) A phenylacetamide compound of the general formula:

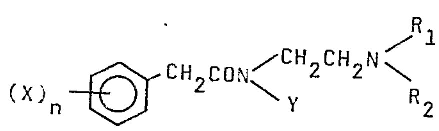

wherein $R_1$ is an alkyl group having 1 to 3 carbon atoms or a hydroxyalkyl group having 2 to 4 carbon atoms, $R_2$ is an alkenyl group having 3 to 6 carbon atoms, a cycloalkyl group having 4 to 7 carbon atoms, a cycloalkylalkyl group having 4 to 7 carbon atoms or tetrahydrofurfuryl group, X is an alkoxy group having 1 to 3 carbon atoms, n is an integer of 0 to 2, Y is

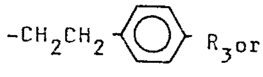 or 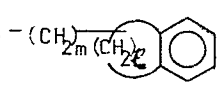

in which $R_3$ is a hydrogen atom, a hydroxyl group or an alkoxyl group having 1 to 3 carbon atoms, m is zero or 1, $\ell$ is an integer of 3 to 5; and the pharmaceutically acceptable salts thereof have a strong analgesic activity and hence are useful as thereapeutically active agents for various types of pain.

EP 0 017 376 A1

TITLE MODIFIED
see front page

This invention relates to phenylacetamide compounds exhibiting strong analgesic activity.

Many compounds which have an acetamide skeleton have been reported to have an analgesic activity in the literature, such as Acta Pharm. Sinica, Vol. 11, pages 108 to 113 (1964), J. Pharm. Sci., Vol. 55, pages 1093 to 1096, (1966), and Austrian Patent No. 221,508. These compounds are amide compounds of phenylacetic acids with anilines, namely, anilides and in these compounds, it has been reported in J. Pharm. Sci, Vol. 55, pages 865 to 887, (1966), J. Med. Chem. Vol. 17, pages 721 to 752, (1964) that compounds wherein the nitrogen atom of the amide group is connected through a hydrocarbon chain to the benzine ring exhibit decreased analgesic activity compared with the compounds wherein the nitrogen atom of amide group is directly attached to the benzene ring such as anilides.

The present invention resides in one aspect in a phenylacetamide compound of the general formula:

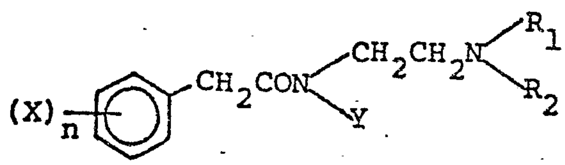

(I)

wherein $R_1$ is an alkyl group having 1 to 3 carbon atoms or a hydroxyalkyl group having 2 to 4 carbon atoms, $R_2$ is an alkenyl group having 3 to 6 carbon atoms, a cycloalkyl group having 4 to 7 carbon atoms, a cycloalkylalkyl group having 4 to 7 carbon atoms or tetrahydrofurfuryl group, X is an alkoxyl group having 1 to 3 carbon atoms, n is an integer of 0 to 2, Y is $-CH_2CH_2-$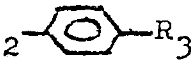$-R_3$ or $-(CH_2)_m$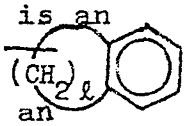 in which $R_3$ is a hydrogen atom, a hydroxyl group or an alkoxyl group having 1 to 3 carbon atoms, m is zero or 1, $\ell$ is an integer of 3 to 5; or a pharmaceutically acceptable salt thereof.

In a further aspect, the invention resides in an analgesic composition comprising a therapeutically effective amount of the compound of formula (I) or a pharmaceutically acceptable salt thereof and one or more pharmaceutically acceptable diluents or carriers.

In yet a further aspect, the invention in a therapeutically effective amount of a compound of formula (I) or a pharmaceutically acceptable salt thereof for use in alleviating pain.

It has been found that the phenylacetamide compounds of the general formula (I):

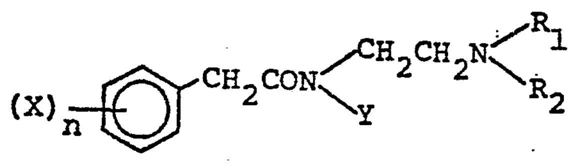

(I)

wherein $R_1$ is an alkyl group having 1 to 3 carbon atoms or a hydroxyalkyl group having 2 to 4 carbon atoms, $R_2$ is an alkenyl group having 3 to 6 carbon atoms, a cycloalkyl group having 4 to 7 carbon atoms, a cycloalkylalkyl group having 4 to 7 carbon atoms or tetrahydrofurfuryl group, X is an alkoxy group having 1 to 3 carbon atoms, n is an integer of 0 to 2, Y is

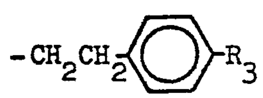 or 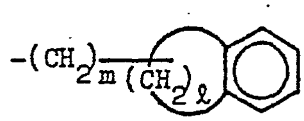 in which $R_3$ is a hydrogen atom, a hydroxyl group or an alkoxy group having 1 to 3 carbon atoms, m is zero or 1, $\ell$ is an integer of 3 to 5, and the pharmaceutically acceptable salts thereof show an inhibitory effect on writhing test induced by acetic acid. As expected from this property, the compounds of formula (I) and their pharmaceutically acceptable salts thereof possess an analgesic action and are useful as therapeutically active agents for various types of pain.

In the compounds of this invention, the nuclear substituent (X) in the phenylacetyl group may be in any position and as that substituent, methoxy, ethoxy and propoxy groups may be employed. Preferably, the X substituent is a methoxy group, and n is preferably one. As the substituent (Y), 2-phenylethyl, 2-(p-methoxyphenyl)-ethyl, 2-(p-ethoxyphenyl)ethyl, 2-(p-propoxyphenyl)ethyl groups and 1- or 2- indanylmethyl, 1- or 2- indanyl groups and 1, 2, 3, 4-terahydro-1 or 2-naphthyl, 1, 2, 3,4-tetrahydro-1 or 2-napththylmethyl groups are preferred, with the 2-phenylethyl group being the most preferred. As the substituent ($R_1$), methyl, ethyl and propyl groups and 2-hydroxyethyl, 3-hydroxypropyl and 4-hydroxybutyl groups may be employed, the preferred examples being ethyl and methyl groups and the most preferred example being a methyl group. An alkenyl group having 3 to 6 carbon atoms, a cycloalkyl group having 4 to 7, a cycloalkylalkyl group having 4 to 7 carbon atoms or a tetrahydrofurfuryl

group is employed in this invention as the substituent ($R_2$). Preferred examples are alkenyl group, cycloalkylalkyl group and tetrahydrofurfuryl group, and most preferred examples are allyl, tetrahydrofurfuryl and cyclopropylmethyl groups.

Compounds carrying as substituents ($R_1$ and $R_2$) of the amino group, di-alkyl groups such as dimethyl, diethyl and dipropyl groups become weak in pharmacological activity.

Of the compounds of this invention, N-2-phenylethyl-N-[2-(N-methyl-N-tetrahydrofurfurylamino)ethyl]-4-methoxyphenylacetamide, N-2-phenylethyl-N-[2-(N-methyl-N-allylamino)ethyl]-4-methoxyphenylacetamide and N-2-phenylethyl-N-[2-(N-methyl-N-cyclopropylmethylamino)ethyl]-4-methoxyphenylacetamide are most preferred.

Compounds of the general formula (I) can be prepared, for example, by a process wherein a phenylacetic acid or a reactive functional derivative of a phenylacetic acid of the general formula (II):

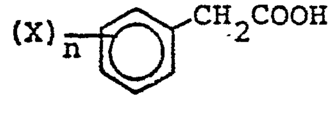

(II)

wherein X and n have the same meaning as given above, is reacted with a compound of the general formula (III):

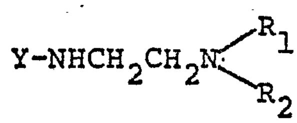

(III)

wherein Y, $R_1$ and $R_2$ have the same meanings as given above, and if desired, hydrolyzing the obtained compound to obtain the end compounds.

When the compound of the general formula (III) above is a compound carrying a hydroxyl group on the benzene nucleus, such compound is preferably protected in the hydroxyl group with benzyl or the like prior to the reaction with a phenylacetic acid of the general formula (II). These protective groups can easily be cleaved in usual manner.

The phenylacetic acid compounds of the general formula (II) above, used as starting materials, can easily be prepared according to methods described in the literature. Examples of such compounds include phenylacetic acid, 2-, 3- or 4-methoxyphenylacetic acid, 2-, 3- or 4-ethoxyphenylacetic acid, 2-, 3- or 4-propoxyphenylacetic acid, 2,3-, 2,4-, 2,5-, 2,6-, 3,4- or 3,5-dimethoxyphenylacetic acid, 2,3-, 2,4-, 2,5-, 2,6-, 3,4- or 3,5-diethoxyphenylacetic acid, 2,3-, 2,4-, 2,5-, 2,6-, 3,4- or 3,5-dipropoxyphenylacetic acid, 3-methoxy-4-ethoxyphenylacetic acid, 3-methoxy-4-propoxyphenylacetic acid, 4-methoxy-3-propoxyphenylacetic acid, 4-methoxy-3-ethoxyphenylacetic acid. In the above process, reactive functional derivatives of such phenylacetic acids are also used as starting materials. Examples of such derivatives include carboxylic acid derivatives, such as acid halides, esters, acid anhydrides, mixed acid anhydrides and a reaction product of such carboxylic acid with a carbodiimide. These reactive functional derivatives can easily be derived from the phenylacetic acids of the general formula (II) according to usual techniques known in this art. For example, the acid chloride can easily be obtained by refluxing for several hours the phenylacetic acid with thionyl chloride in the absence of any solvent or in dry benzene.

The compounds of the general formula (III) above, used as starting materials, can easily be prepared by reacting a compound of the general formula (IV):

$$Y\text{-}NH_2 \qquad (IV)$$

wherein Y has the same meanings as given above, with chloroacetyl chloride in aqueous dioxane in the presence of sodium bicarbonate, and reacting the obtained compound of the general formula (V):

$$Y\text{-}NH\text{-}COCH_2Cl \qquad (V)$$

wherein Y has the same meaning as given above, with a compound of the general formula (VI):

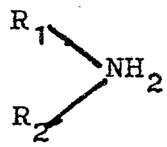

(VI)

wherein $R_1$ and $R_2$ have the same meaning as given above, and then hydrogenating the resulting compound of general formula (VII):

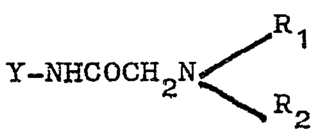

(VII)

wherein Y, $R_1$ and $R_2$ have the same meaning as given above, using aluminum lithium hydride. Alternatively, a compound of the general formula (V) above can be reacted with a compound of the general formula (VIII)

$$R_1NH_2 \qquad (VIII)$$

wherein $R_1$ has the same meaning as given above, or a compound of the general formula (IX):

$$R_2NH_2 \qquad (IX)$$

wherein $R_2$ has the same meaning as given above, and the obtained compound can be treated with a compound of the general formula (X):

$$R_2Z \quad (X)$$

wherein Z is a halogen atom, $R_2$ has the same meaning as given above, or a compound of the general formula (XI):

$$R_1Z \quad (XI)$$

wherein Z and $R_1$ has the same meaning as given above, and then the obtained compound can be hydrogenated using aluminum lithium hydride to obtain the compound of the general formula (III) above.

Examples of the compounds of the general formula (III) above include N-2-phenylethyl-N'-methyl-N'-allyl-ethylenediamine, N-2-phenylethyl-N'-ethyl-N'-allylethyl-enediamine, N-2-phenylethyl-N'-propyl-N'-allylethylene-diamine, N-2-phenylethyl-N'-methyl-N'-(3,3-dimethylallyl)ethylenediamine, N-2-phenylethyl-N'-ethyl-N'-(3,3-dimethylallyl)ethylenediamine, N-2-phenylethyl-N'-propyl-N'-(3,3-dimethylallyl)ethylenediamine, N-2-(4-methoxyphenyl)ethyl-N'-methyl-N'-(3,3-dimethylallyl)ethylenediamine, N-2-(4-methoxyphenyl)ethyl-N'-ethyl-N'-(3,3-dimethylallyl)ethylenediamine,

N-2-(4-methoxyphenyl)ethyl-N'-propyl-N'-(3,3-dimethylallyl)ethylenediamine, N-2-phenylethyl-N'-(2-hydroxyethyl)-N'-allyethylenediamine, N-2-phenylethyl-N'-methyl-N'-tetrahydrofurfurylethylenediamine, N-2-phenylethyl-N'-ethyl-N'-tetrahydrofurfurylethylenediamine, N-2-phenylethyl-N'-propyl-N'-tetrahydrofurfurylethylenediamine, N-2-(4-methoxyphenyl)ethyl-N'-methyl-N'-tetrahydrofurfurylethylenediamine, N-2-(4-methoxyphenyl)ethyl-N'-ethyl-N'-tetrahydrofurfurylethylenediamine, N-2-(4-methoxyphenyl)ethyl-N'-propyl-N'-tetrahydrofurfurylethylenediamine, N-2-(4-ethoxyphenyl)ethyl-N'-methyl-N'-tetrahydrofurfurylethylenediamine, N-2-(4-ethoxyphenyl)ethyl-N'-ethyl-N'-tetrahydrofurfurylethylenediamine, N-2-(4-ethoxyphenyl)ethyl-N'-propyl-N'-tetrahydrofurfurylethylenediamine, N-2-(4-propoxyphenyl)-ethyl-N'-methyl-N'-tetrahydrofurfurylethylenediamine, N-2-(4-propoxyphenyl)ethyl-N'-ethyl-N'-tetrahydrofurfurylethylenediamine, N-2-(4-propoxyphenyl)ethyl-N'-propyl-N'-tetrahydrofurfurylethylenediamine, N-2-phenylethyl-N'-methyl-N'-cyclopropylmethylethylenediamine, N-2-phenylethyl-N'-ethyl-N'-cyclopropylmethylethylenediamine, N-2-phenylethyl-N'-propyl-N'-cyclopropylmethylethylenediamine, N-2-phenylethyl-N'-methyl-N'-cyclohexylethylenediamine, N-2-phenylethyl-N'-ethyl- N'-cyclohexylethylenediamine, N-2-phenylethyl-N'-

propyl-N'-cyclohexylethylenediamine, N-2-(4-methoxyphenyl)ethyl-N'-methyl-N'-cyclopropylmethylethylenediamine, N-2-(4-methoxyphenyl)ethyl-N'-methyl-N'-cyclohexylethylenediamine, N-2-(4-methoxyphenyl)ethyl-N'-ethyl-N'-cyclopropylmethylethylenediamine, N-2-(4-methoxyphenyl)ethyl-N'-ethyl-N'-cyclohexylethylenediamine, N-2-(4-methoxyphenyl)ethyl-N'-propyl-N'-cyclopropylmethylethylenediamine, N-2-(4-methoxyphenyl)-ethyl-N'-propyl-N'-cyclohexylethylenediamine, N-2-(4-ethoxyphenyl)ethyl-N'-methyl-N'-cyclopropylmethyl-ethylenediamine, N-2-(4-ethoxyphenyl)ethyl-N'-ethyl-N'-cyclohexylethylenediamine, N-2-(4-ethoxyphenyl)ethyl-N'-ethyl-N'-cyclopropylmethylethylenediamine, N-2-(4-propoxyphenyl)ethyl-N'-propyl-N'-cyclopropylmethyl-ethylenediamine, N-2-(4-benzyloxyphenyl)ethyl-N'-methyl-N'-cyclopropylmethylethylenediamine, N-2-(4-benzyloxyphenyl)ethyl-N'-methyl-N'-cyclohexylethylenediamine, N-2-(4-benzyloxyphenyl)ethyl-N'-ethyl-N'-cyclopropylmethylethylenediamine, N-2-(4-benzyloxyphenyl)ethyl-N'-ethyl-N'-cyclohexylethylenediamine, N-2-(4-benzyloxyphenyl)ethyl-N'-propyl-N'-cyclohexylethylenediamine, N-2-phenylethyl-N'-methyl-N'-cyclobutyl-ethylenediamine, N-2-phenylethyl-N'-ethyl-N'-cyclobutylethylenediamine, N-2-phenylethyl-N'-propyl-N'-cyclo-

butylethylenediamine, N-2-(4-methoxyphenyl)ethyl-N'-methyl-N'-cyclobutylethylenediamine, N-2-(4-methoxyphenyl)ethyl-N'-ethyl-N'-cyclobutylethylenediamine, N-(1,2,3,4-tetrahydro-1-naphthylmethyl)-N'-methyl-N'-allylethylenediamine, N-(1,2,3,4-tetrahydro-1-naphthylmethyl)-N'-methyl-N'-(3,3-dimethylallyl)ethylenediamine, N-(1,2,3,4-tetrahydro-1-naphtyhlmethyl)-N'-methyl-N'-tetrahydrofurfurylethylenediamine, N-(1,2,3,4-tetrahydro-2-naphthyl)-N'-methyl-N'-allylethylenediamine, N-(1,2,3,4-tetrahydro-2-naphthyl)-N'-methyl-N'-(3,3-dimethylallyl)ethylenediamine, N-(1,2,3,4-tetrahydro-2-naphthyl)-N'-methyl-N'-tetrahydrofurfurylethylenediamine, N-(1,2,3,4-tetrahydo-1-naphthylmethyl)-N'-methyl-N'-cyclopropylmethylethylenediamine, N-(1,2,3,4-tetrahydro-2-naphthyl)-N'-methyl-N'-cyclopropylmethylethylenediamine, N-2-indanyl-N'-methyl-N'-allylethylenediamine, N-2-indanyl-N'-methyl-N'-(3,3-dimethylallyl)ethylenediamine, N-2-indanyl-N'-methyl-N'-tetrahydrofurfurylethylenediamine, N-2-indanyl-N'-methyl-N'-cyclopropylmethylethylenediamine, N-1-indanylmethyl-N'-methyl-N'-allylethylenediamine, N-1-indanylmethyl-N'-methyl-N'-(3,3-dimethylallyl)ethylenediamine, N-1-indanylmethyl-N'-methyl-N'-tetrahydrofurfurylethylenediamine, N-1-indanylmethyl-N'-methyl-N'-cyclopropylmethylethylenediamine, etc.

The amidation described above in the reaction of the compound of the general formula (II) with the compound of the general formula (III) can be carried out according to methods known per se. For example, when the phenylacetic acid compound of the general formula (II) is used as a starting material, a mixture of the phenylacetic acid compound and the compound of the general formula (III) above in a proportion of about 1 to 0.8 mols per mol of the phenylacetic acid compound is heated to about 50 to about 200°C, preferably 120 to 180°C, for about 10 to about 24 hours, preferably 15 to 20 hours in an inert organic solvent such as xylene or toluene Alternatively, a mixture of the phenylacetic acid compound and the compound of the general formula (III) in proportion of about 1 to about 0.8 mols per mol of the phenylacetic acid compound in an inert organic solvent such as ethers, e.g., dioxane, tetrahydrofuran, etc., aromatic hydrocarbons, benzene, xylene, toluene, halogenated hydrocarbons, e.g., dichloromethane, dichloroethane, chloroform, etc., and an adequate amount of a condensing agent is added to the mixture with ice-cooling and stirring, and then the mixture is stirred at room temperature or at about 50 to about 150°C, preferably 50 to 120°C for about 1 to about 10 hours, preferably 2 to 5 hours, or the compound of

the general formula (III) is added to a mixture of the phenylacetic acid compound in a proportion of about 1 to about 1.2 mols per mol of the compound of the general formula (III) and an adequate amount of a condensing agent in an inert organic solvent such as those described above with cooling and stirring, and then the mixture is stirred at room temperature or at about 50 to about 150°C, preferably 50 to 120°C, for about 1 to 10 hours, preferably 2 to 5 hours.

A suitable amount of the condensing agent which can be used in this process is from about 1 to about 1.3 mols, preferably 1 mol, per mol of the compound of the general formula (II).

Suitable examples of the condensing agent which can be used in this process include phosphorus oxychloride, phosphorus pentoxide, phosphorus trichloride, phosphorus tribromide, phosphorus pentabromide, polyphosphoric acid, polyphosphoric acid esters, methyl dichlorophosphite, dimethyl chlorophosphite, diethyl chlorophosphite, ethylene chlorophosphite, phenylene chlorophosphate, ethyl dichlorophosphate, phenyl dichlorophosphine oxide, dibenzyl chlorophosphine oxide, and tetraethyl pyrophosphite.

When a reactive functional derivative of the phenylacetic acid compound is used as a starting material, for example, the acid chloride is reacted with the compound of the general formula (III) above in a proportion of about 1 to about 0.8 mols per mol of the phenylacetic acid halide of the general formula (II) above at about 0 to about 100°C, preferably room temperature to 70°C, for

about 1 to 5 hours, preferably 2 to 3 hours, in an inert solvent, such as diethyl ether and water as well as those described above. This procedure is preferably carried out by dissolving a compound of the general formula (III) in an inert solvent together with an adequate amount of a basic substance with cooling and stirring, adding a solution of a slightly excess amount of or an equimolar amount of an acid halide of the phenylacetic acid compound in an inert solvent and refluxing the mixture for several hours.

In this case, suitable examples of the basic substance include a tertiary organic base such as triethylamine or pyridine, or an inorganic base such as caustic alkali, sodium carbonate or potassium carbonate. This reaction can also be carried out using 4-(N,N-dimethylamine) pyridine as catalyst.

The reaction product is washed with an alkaline

solution, for example, an aqueous solution of about 5 to about 10% by weight of an alkali metal hydroxide, such as NaOH, KOH, LiOH, etc., an alkaline earth metal hydroxide such as $Ca(OH)_2$, $Mg(OH)_2$, etc., then extracted with an inert solvent such as ether.

The resultant solvent layer is then dried, and concentrated under reduced pressure. The residue is distilled under reduced pressure to obtain the end product. Alternatively, the washed reaction mixture is concentrated under reduced pressure and the residue is purified by column-chromatography using active alumina to obtain the end product.

The compound of this invention in the free form is a viscous oily substance and is soluble in usual organic solvents, such as chloroform, alcohols, e.g., methanol, ethanol, etc., esters, e.g., ethyl acetate, methyl acetate, etc., ethers, e.g., dimethyl ether, diethyl ether, tetrahydrofuran, dioxane, etc.

The resulting compound of the general formula (I) having a free-amino group can be converted according to usual methods to pharmaceutically acceptable acid-addition salts thereof. For example, the compound of the general formula (I) is dissolved in diethylether, and hydrogen chloride in an equimolar or excess amount

is bubbled into the ether solution, then the precipitated crystals are collected by filtration and, if desired, are recrystallized from a suitable solvent whereby the compound can be converted into its hydrochloric acid salt. Suitable examples of such pharmaceutically acceptable salts include, in addition to hydrochloric acid salts, sulfonic acid salts hydrobromic acid salts, citric acid salts, succinic acid salts, and lactic acid salts. These salts are crystalline substances or gelatin-like semi-solid substances having glassy appearance, soluble in water and alcohols.

These pharmaceutically acceptable acid-addition salts of the compounds of this invention have as high a pharmacological effect as the corresponding compounds containing free-amino group.

The phenylacetamide compounds of this invention possess strong analgesic activity, and are useful as anodynes.

The compound of the general formula (I) and the pharmaceutically acceptable salts thereof of the present invention can be administered to mammals including humans by oral, intravenous, intramuscular or intrarectal administration, and can be formulated into pharmaceutical compositions together

with ordinary pharmaceutically acceptable carriers.

Various dosage forms of the therapeutic agents as an analgesic agent can be selected according to the purpose of the therapy. Typical dosage forms which can be used are tablets, pills, powders, liquid preparations, suspensions, emulsions, granules, capsules, suppositories, and injectable preparations.

In molding the pharmaceutical composition into a tablet form, a wide variety of conventional carriers known in the art can be used. Examples of suitable carriers are excipients, such as glucose, lactose, starch, cacao butter, hardened vegetable oils, kaolin and talc, binders, such as gum arabic powder, tragacanth powder, gelatin, and ethanol, and disintegrants, such as laminaria and agar. The tablets, if desired, can be coated, and made into sugar-coated tablets, gelatin-coated tablets, enteric-coated tablets, film-coated tablets, or tablets coated with two or more layers.

When the pharmaceutical composition is formulated into an injectable preparation, the resulting solution and suspension are preferably sterilized, and are isotonic with respect to the blood. In formulating the pharmaceutical composition into the form of a solution or suspension, all diluents customarily used

in the art can be used. Examples of suitable diluents are water, ethyl alcohol, propylene glycol, ethoxylated isostearyl alcohol, polyoxyethylene sorbitol, and sorbitan esters. Sodium chloride, glucose or glycerol may be incorporated into an analgesic agent in an amount sufficient to prepare isotonic solutions. The therapeutic agent may further contain ordinary dissolving acids, buffers, pain-alleviating agents, and preservatives, and optionally coloring agents, perfumes, flavors, sweeteners, and other drugs.

The dosage of the compound of this invention is suitably selected according to the purpose of use, the symptoms, etc. Usually, a preferred dosage of the compound of this invention is about 0.5 to 10 mg/kg of body weight per day in multiple doses.

This invention is further illustrated in more detail by way of examples wherein the melting points or the boiling points of the products obtained are uncorrected. Unless otherwise indicated, all parts, percents, ratios and the like are by weight.

REFERENCE EXAMPLE 1

To an aqueous solution of 59.5 g of 2-(4-methoxyphenyl)ethylamine hydrochloride and 120 g of sodium bicarbonate in 800 ml of dioxane and 1000 ml of water

was added dropwise 60 g of chloroacetyl chloride with ice-cooling and stirring and then the mixture was stirred for 2 hours at room temperature. The reaction mixture was concentrated under reduced pressure and an adequate amount of water was added to the residue, and then the precipitate was collected and recrystallized from chloroform-n-hexane to obtain 50.5 g of N-[2-(4-methoxyphenyl)ethyl]chloroacetamide.

A mixture of 8 g of N-[2-(4-methoxyphenyl)ethyl]-chloroacetamide and 50 g of a 40% methanol solution of methylamine was heated for 4 hours at 100°C in a sealed tube. After cooling, the reaction mixture was concentrated under reduced pressure and an adequate amount of a 10% aqueous solution of sodium hydroxide was added to the residue to make the liquid alkali, and then the alkaline mixture was extracted with benzene. The benzene layer was washed with water, dried and concentrated under reduced pressure to obtain 7.5 g of N-[2-(4-methoxyphenyl)ethyl]methylaminoacetamide.

A mixture of 7.5 g of N-[2-(4-methoxyphenyl)ethyl]-methylaminoacetamide, 4 g of 3,3-dimethylallyl chloride, 13 g of potassium carbonate and 0.5 g of sodium iodide in 140 ml of ethanol and 50 ml of water was heated under reflux for 5 hours. The reaction mixture was concentrated under reduced pressure and an adequate

amount of a 10% aqueous solution of sodium hydroxide was added to the residue to make the liquid alkali, and then the alkaline mixture was extracted with benzene. The benzene layer was washed with water, dried and concentrated under reduced pressure to obtain 6.8 g of N-2-(4-methoxyphenyl)ethyl-[N-methyl-N-(3,3-dimethylallyl)amino]acetamide

To a suspension of 5.3 g of aluminum lithium hydride in 50 ml of ether was added dropwise 6.8 g of N-2-(4-methoxyphenyl)ethyl-[N-methyl-N-(3,3-dimethylallyl)-amino]acetamide with ice-cooling and stirring, and the mixture was stirred for 1 hour at room temperature and then heated under reflux overnight. After cooling, an adequate amount of a 10% aqueous solution of sodium hydroxide was added dropwise to the reaction mixture with ice-cooling and stirring until the evolution of hydrogen gas ceased. The mixture was filtered and an organic phase was separated, dried and concentrated under reduced pressure. The residue was distilled under reduced pressure (130-135°C/0.5 mmHg) to obtain 4.9 g of N-2-(4-methoxyphenyl)ethyl-N'-methyl-N'-(3,3-dimethylallyl)ethylenediamine.

IR-absorption spectrum (neat)

$\nu$NH: 3300 $cm^{-1}$

Elementary analysis as $C_{17}H_{28}N_2O$

| | C% | H% | N% |
|---|---|---|---|
| Calcd. | 73.86 | 10.21 | 10.14 |
| Found | 73.73 | 10.18 | 10.21 |

NMR spectrum ($CDCl_3$)

δ: 1.65 (s, 3H), 1.75 (s, 3H), 2.20 (s, 3H), 2.3-3.1 (m, 11H), 3.80 (s, 3H), 5.1-5.4 (m, 1H), 6.80 (d, 2H), 7.14 (d, 2H),

REFERENCE EXAMPLE 2

To a solution of 38 g of 2-phenylethylamine hydrochloride and 80 g of sodium bicarbonate in 600 ml of dioxane and 700 ml of water was added dropwise 40 g of chloroacetyl chloride with ice-cooling and stirring and the mixture was stirred for 2 hours at room temperature. The reaction mixture was concentrated under reduced pressure and an adequate amount of water was added to the residue, and then the precipitate was collected and recrystallized from chloroform-n-hexane to obtain 36.2 g of N-(2-phenylethyl)chloroacetamide.

A mixture of 36.2 g of N-(2-phenylethyl)chloroacetamide, 22.2 g of N-methylcyclopropylmethylamine hydrochloride and 40.7 g of triethylamine in 200 ml of benzene was heated under reflux overnight. After cooling, an adequate amount of a 10% aqueous solution

of sodium hydroxide was added to the reaction mixture to make the liquid alkaline and an organic phase was separated, washed with water, dried and concentrated under reduced pressure to obtain 45.3 g of N-2-phenylethyl-[(N-methyl-N-cyclopropylmethyl)amino]acetamide.

To a suspension of 14 g of aluminum lithium hydride in 1000 ml of ether was added dropwise a solution of 45.3 g of N-2-phenylethyl-[(N-methyl-N-cyclopropylmethyl)-amino]acetamide in 300 ml of ether with ice-cooling and stirring, and the mixture was stirred for 1 hour at room temperature and then heated under reflux overnight. After cooling, an adequate amount of a 10% aqueous solution of sodium hydroxde was added to the reaction mixture with ice-cooling and stirring until the evolution of hydrogen gas ceased. The mixture was filtered and an organic phase separated, dried and concentrated under reduced pressure. The residue was distilled under reduced pressure (130-135°C/1 mmHg) to obtain 31.9 g of N-2-phenylethyl-N'-methyl-N'-cyclopropylmethylethylene-diamine.

IR-absorption spectrum (neat)

$\nu$NH: 3300 $cm^{-1}$

Elementary analysis as $C_{15}H_{24}N_2$

| | C% | H% | N% |
|---|---|---|---|
| Calcd. | 77.53 | 10.41 | 12.06 |
| Found | 77.46 | 10.46 | 11.98 |

NMR spectrum ($CDCl_3$)

δ: 0-1.05 (m, 5H), 1.8 (s, 1H), 2.15-3.0 (m, 13H), 7.2 (s, 5H)

The following compounds can be prepared in a similar manner. The results obtained are shown in Table 1 below.

Table 1.

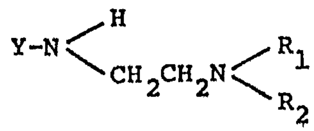

| Com. No. | Y | $R_1$ | $R_2$ | boiling point | liquid condition | IR-absorption spectrum |
|---|---|---|---|---|---|---|
| 1 | 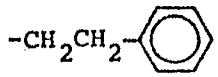 | $-CH_3$ | $-CH_2CH=CH_2$ | 115-120°C (1 mmHg) | pale yellow oil | νNH:3300 $cm^{-1}$ |
| 2 | 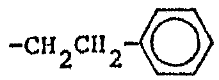 | $-CH_3$ | $-CH_2CH=C(CH_3)_2$ | 120-123°C (1 mmHg) | colorless oil | νNH:3300 $cm^{-1}$ |
| 3 | 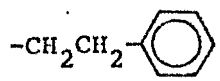 | $-CH_2CH_2OH$ | $-CH_2CH=CH_2$ | 110-115°C (1 mmHg) | colorless oil | νOH:3250 $cm^{-1}$ |
| 4 | 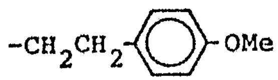 | $-CH_3$ | 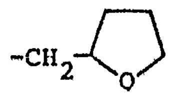 | 204-207°C (3 mmHg) | colorless oil | νNH:3300 $cm^{-1}$ |
| 5 | 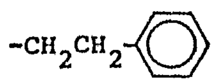 | $-CH_3$ | 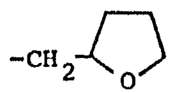 | 165-166°C (3 mmHg) | colorless oil | νNH:3300 $cm^{-1}$ |
| 6 | 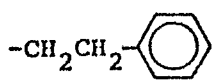 | $-CH_2CH_2CH_3$ | 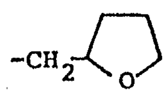 | 187-189°C (4 mmHg) | pale yellow oil | νNH:3300 $cm^{-1}$ |

| Com. No. | Y | $R_1$ | $R_2$ | boiling point | liquid condition | IR-absorption spectrum |
|---|---|---|---|---|---|---|
| 7 | 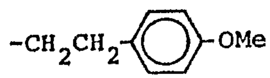 | $-CH_3$ | 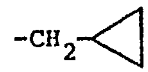 | 135-140°C (1 mmHg) | colorless oil | νNH:3300 $cm^{-1}$ |
| 8 | 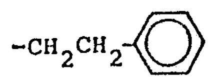 | $-CH_3$ | 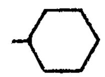 | 163-165°C (5 mmHg) | colorless oil | νNH:3300 $cm^{-1}$ |
| 9 | 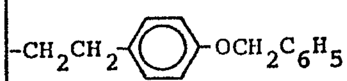 | $-CH_3$ | 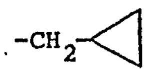 | * | pale yellow oil | νNH:3300 $cm^{-1}$ |
| 10 | 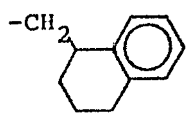 | $-CH_3$ | $-CH_2CH=C(CH_3)_2$ | 160-165°C (1 mmHg) | colorless oil | νNH:3300 $cm^{-1}$ |
| 11 | 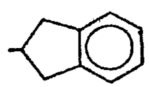 | $-CH_3$ | 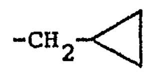 | 143-145°C (1 mmHg) | colorless oil | νNH:3290 $cm^{-1}$ |
| 12 | 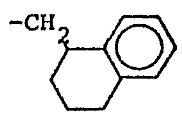 | $-CH_3$ | 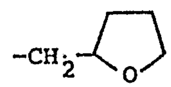 | 195-200°C (2 mmHg) | colorless oil | νNH:3300 $cm^{-1}$ |
| 13 | 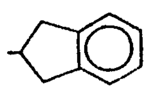 | $-CH_3$ | 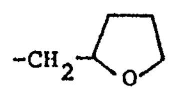 | 168-170°C (2 mmHg) | pale yellow oil | νNH:3300 $cm^{-1}$ |

* The compounds were purified by column-chromatography using active alumina and a mixture of benzene and ethyl acetate as eluent.

EXAMPLE 1

To a solution of 3 g of N-2-phenylethyl-N'-(2-hydroxyethyl)-N'-allylethylenediamine and 10 g of triethylamine in 50 ml of benzene was added dropwise a solution of 7 g of 4-methoxyphenylacetyl chloride in 50 ml of benzene with ice-cooling and stirring, and the mixture was stirred for 1 hour at room temperature and then heated under reflux for 3 hours. After cooling, the reaction mixture was washed successively with a 10% aqueous solution of sodium hydroxide and water, dried and concentrated under reduced pressure. The residue was dissolved in 50 ml of ethanol containing 2 g of sodium hydroxide and the solution was stirred overnight at room temperature. The reaction mixture was concentrated under reduced pressure, and an adequate amount of water was added to the residue and the mixture was extracted with benzene. The benzene layer was washed with water, dried and concentrated under reduced pressure. The residue was purified by column-chromatography using active alumina and a mixture of benzene and ethyl acetate as eluent to obtain 3 g of N-2-phenylethyl-N-[2-(N-2-hydroxyethyl-N-allylamino)ethyl]-4-methoxyphenylacetamide. (pale yellow oil)

IR-absorption spectrum (neat)

$\nu$CO: 1640 $cm^{-1}$

$\nu$OH: 3400 $cm^{-1}$

Elementary analysis as $C_{24}H_{32}N_2O_3$

| | C% | H% | N% |
|---|---|---|---|
| Calcd. | 72.69 | 8.13 | 7.07 |
| Found | 72.73 | 8.25 | 6.92 |

NMR spectrum ($CDCl_3$)

δ: 2.4-7.7 (m, 17H), 3.72 (s, 3H), 5.0-5.3 (m, 2H), 5.5-6.0 (m, 1H), 6.7-7.4 (m, 9H)

EXAMPLE 2

To a solution of 3.4 g of 4-methoxyphenylacetic acid in 80 ml of benzene was added dropwise 3.2 g of phosphorus oxychloride with ice-cooling and stirring and the mixture was stirred for 30 minutes at room temperature, and then 3 g of N-2-phenylethyl-N'-methyl-N'-allylethylenediamine was added to the mixture and then the mixture was heated under reflux for 3 hours. After completion of the reaction, an adequate amount of a 10% aqueous solution of sodium hydroxide was added to the reaction mixture to make the liquid alkaline The alkaline mixture was extracted with benzene, and the benzene layer was washed with water, dried and concentrated under reduced pressure. The residue was distilled under reduced pressure (170-180°C/0.5 mmHg) to obtain 4.5 g of N-2-phenylethyl-N-[2-(N-methyl-N-allylamino)-ethyl]-4-methoxyphenylacetamide as a pale yellow

substance.

IR-absorption spectrum (neat)

νCO: 1640 $cm^{-1}$

Elementary analysis as $C_{23}H_{30}N_2O_2$

| | C% | H% | N% |
|---|---|---|---|
| Calcd. | 75.37 | 8.25 | 7.64 |
| Found | 75.27 | 8.45 | 7.39 |

NMR spectrum ($DMSO-d_6$)

δ: 2.15 (s, 3H), 2.2-3.7 (m, 12H), 3.75 (s, 3H), 4.9-5.3 (m, 2H), 5.5-6.0 (m, 1H), 6.7-7.3 (m, 9H)

EXAMPLE 3

To a solution of 7.9 g of N-2-phenylethyl-N'-methyl-N'-tetrahydrofurfurylethylenediamine, 10 ml of triethylamine and 190 mg of 4-(N,N-dimethylamino)pyridine in 100 ml of dichloromethane was added dropwise a solution of 6.5 g of 4-methoxyphenylacetyl chloride in 20 ml of dichloromethane with ice-cooling and stirring and the mixture was stirred for 1 hour at room temperature. After completion of the reaction, an adequate amount of a 10% aqueous solution of sodium hydroxide was added to the reaction mixture to make the liquid alkaline. An organic phase was separated, washed with water, dried and concentrated under reduced pressure. The

residue was distilled under reduced pressure (243-245°C/0.2 mmHg) to obtain 9 g of N-2-phenylethyl-N-[2-(N-methyl-N-tetrahydrofurfurylamino)ethyl]-4-methoxyphenylacetamide.

IR-Absorption spectrum (neat)

νCO: 1635 $cm^{-1}$

Elementary analysis as $C_{25}H_{34}N_2O_3$

| | C% | H% | N% |
|---|---|---|---|
| Calcd. | 73.14 | 8.35 | 6.82 |
| Found | 73.09 | 8.15 | 6.89 |

NMR spectrum ($CDCl_3$)

δ: 1.2-4.15 (m, 22H), 3.77 (s, 3H), 6.7-7.4 (m, 9H)

EXAMPLE 4

To a solution of 5 g of 3,4-dimethoxyphenylacetic acid in 20 ml of benzene was added dropwise 4 g of phosphorus oxychloride with ice-cooling and stirring and the mixture was stirred for 30 minutes at room temperature. A solution of 4.6 g of N-2-phenylethyl-N'-methyl-N'-terahydrofurfurylethylenediamine in 20 ml of benzene was added to the mixture with ice-cooling and stirring and the mixture was stirred for 30 minutes at room temperature and then heated under reflux for 3 hours. After completion of the reaction, an adequate

amount of a 10% aqueous solution of sodium hydroxide was added to the reaction mixture to make the liquid alkaline. An organic phase was separated, washed with water, dried and concentrated under reduced pressure. The residue was purified by column-chromatography using active alumina and a mixture of benzene and ethyl acetate as eluent to obtain 5.3 g of N-2-phenylethyl-N-[2-(N-methyl-N-tetrahydrofurfuryamino)ethyl]-3,4-dimethoxyphenylacetamide. (pale yellow oil)

IR-absorption spectrum (neat)

νCO: 1640 $cm^{-1}$

Elementary analysis as $C_{26}H_{36}N_2O_4$

| | C% | H% | N% |
|---|---|---|---|
| Calcd. | 70.88 | 8.24 | 6.36 |
| Found | 70.97 | 8.22 | 6.21 |

NMR spectrum ($CDCl_3$)

δ: 1.30-4.0 (m, 22H), 3.85 (s, 3H), 3.88 (s, 3H), 6.6-7.4 (m, 8H)

EXAMPLE 5

To a solution of 5 g of 4-methoxyphenylacetic acid in 30 ml of benzene was added dropwise 6 g of phosphorus oxychloride with ice-cooling and stirring and the mixture was stirred for 30 minutes at room temperature. A solution of 6 g of N-(1,2,3,4-tetrahydro-1-naphthylmethyl)-N'-methyl-N'-tetrahydrofurfurylethylenediamine in 20 ml of benzene was added dropwise to the mixture. The mixture was stirred for 1 hour at room temperature and then heated under reflux for 3 hours. After cooling, an adequate amount of a 10% aqueous solution of sodium hydroxide was added to the reaction mixture to make the liquid alkaline and the alkaline mixture was extracted with benzene, washed with water, dried and concentrated under reduced pressure. The residue was purified by column-chromatography using active alumina and a mixture of benzene and ethyl acetate as eluent to obtain 6.5 g of N-(1,2,3,4-tetrahydro-1-naphthylmethyl)-N-[2-(N-methyl-N-tetrahydrofurfurylamino)ethyl]-4-methoxyphenylacetamide. (yellow oil)

IR-absorption spectrum (neat)

νCO: 1640 $cm^{-1}$

Elementary analysis as $C_{28}H_{38}N_2O_3$

| | C% | H% | N% |
|---|---|---|---|
| Calcd. | 74.63 | 8.50 | 6.22 |
| Found | 74.50 | 8.45 | 5.95 |

NMR-spectrum ($CDCl_3$)

δ: 1.0-4.1 (m, 27H), 3.78 (s, 3H), 6.6-7.3 (m, 8H)

EXAMPLE 6

To a solution of 2 g of N-2-indanyl-N'-methyl-N'-cyclopropylmethylethylenediamine, 50 mg of 4-(N,N-dimethylamino)pyridine and 3 g of triethylamine in 30 ml of dichloromethane was added dropwise a solution of 1.6 g of phenylacetyl chloride in 5 ml of dichloromethane with ice-cooling and stirring. The mixture was stirred for 2 hours at room temperature. The reaction mixture was concentrated under reduced pressure and an adequate amount of a 10% aqueous solution of sodium hydroxide was added to the residue to make the liquid alkaline. The alkaline mixture was extracted with benzene, and the benzene layer was washed with water, dried and concentrated under reduced pressure. The residue was purified by column-chromatography using active alumina and a mixture of benzene and ethyl acetate as eluent to obtain 1.8 g of N-2-indanyl-N-[2-(N-methyl-N-cyclopropylmethyl)aminoethyl]phenylacetamide. (pale yellow oil)

IR-absorption spectrum (neat)

νCO: 1640 $cm^{-1}$

Elementary analysis as $C_{24}H_{30}N_2O$

| | C% | H% | N% |
|---|---|---|---|
| Calcd. | 79.51 | 8.34 | 7.73 |
| Found | 79.39 | 8.29 | 7.81 |

NMR spectrum ($CDCl_3$)

δ: 0-1.0 (m, 5H), 2.0-4.0(m, 14H), 3.80 (s, 2H), 7.0-7.5 (m, 9H)

EXAMPLE 7

To a solution of 10 g of N-2-(4-benzyloxyphenyl)-ethyl-N'-methyl-N'-cyclopropylmethylethylenediamine and 8.3 g of 4-methoxyphenylacetic acid in 100 ml of benzene was added dropwise 7.7 g of phosphorus oxychloride with ice-cooling and stirring. The mixture was stirred for 1 hour at room temperature and then heated under reflux for 5 hours. After completion of the reaction, an adequate amount of a 10% aqueous solution of sodium hydroxide was added to the reaction mixture to make the liquid alkaline and the alkaline mixture was extracted with benzene. The benzene layer was washed with water, dried and concentrated under reduced pressure, and the residue was purified by column-chromatography using active alumina and a mixture of benzene and ethyl acetate as eluent to obtain 10 g of N-2-(4-benzyloxyphenyl)ethyl-N-[2-(N-methyl-N-cyclopropylmethyl)aminoethyl]-4-methoxyphenylacetamide.

A solution of 10 g of N-2-(4-benzyloxyphenyl)ethyl-N-[2-(N-methyl-N-cyclopropylmethyl)aminoethyl]-4-methoxyphenylacetamide, 3 ml of concentrated hydrochloric acid and 300 ml of ethanol was hydrogenated over 0.6 g

of palladium chloride at room temperature under 4 atms. The reaction mixture was filtered and the filtrate was concentrated under reduced pressure.

A solution of 9 g of diethylamine in 500 ml of diethylether was added to the residue and the precipitate was filtered off, and the filtrate was concentrated under reduced pressure to obtain 4.8 g of N-2-(4-hydroxyphenyl)ethyl-N-[2-(N-methyl-N-cyclopropylmethyl)aminoethyl]-4-methoxyphenylacetamide as a pale yellow oil.

IR-absorption spectrum (neat)

νOH: 3250 $cm^{-1}$

νCO: 1630 $cm^{-1}$

Elementary analysis as $C_{24}H_{32}N_2O_3$

| | C% | H% | N% |
|---|---|---|---|
| Calcd. | 72.69 | 8.13 | 7.07 |
| Found | 72.51 | 8.21 | 6.87 |

NMR spectrum ($CDCl_3$)

δ: 0-1.1 (m, 5H), 2.2-3.9 (m, 15H), 3.76 (s, 3H), 6.6-7.3 (m, 8H), 8.38 (br, 1H)

EXAMPLE 8

To a solution of 3.5 g of N-2-phenylethyl-N'-methyl-N'-cyclopropylmethylethylenediamine, 5 ml of triethylamine and 20 mg of 4-(N,N-dimethylamino)pyridine in 50 ml of dichloromethane was added dropwise a solution

of 3.5 g of 2,5-dimethoxyphenylacetyl chloride in 10 ml of dichloromethane with ice-cooling and stirring. The mixture was stirred for 1 hour under ice-cooling and then stirred for 2 hours at room temperature. After compeltion of the reaction, an adequate amount of an aqueous solution of sodium hydroxide was added to the reaction mixture to make the liquid alkaline and an oraganic phase was separated, washed with water, dried and concentrated under reduced pressure. The residue was purified by column-chromatography using active alumina and a mixture of benzene and ethyl acetate as eluent to obtain 4.6 g of N-2-phenylethyl-N-[2-(N-methyl-N-cyclopropylmethyl)aminoethyl]-2,5-dimethoxyphenylacetamide.

IR-absorption spectrum (neat)

νCO: 1640 $cm^{-1}$

Elementary analysis as $C_{25}H_{34}N_2O_3$

| | C% | H% | N% |
|---|---|---|---|
| Calcd. | 73.14 | 8.35 | 6.82 |
| Found | 73.31 | 8.44 | 6.83 |

NMR spectrum ($CDCl_3$)

δ: 0-1.1 (m, 5H), 2.1-3.9 (m, 15H), 3.73 (s, 6H), 6.70-7.45 (m, 8H)

The following compounds can be prepared in a similar manner. The results obtained are shown in Table 2 below.

Table 2

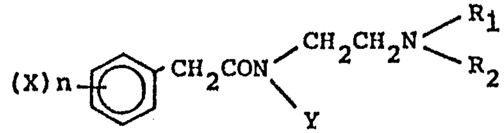

| Com. No. | X | n | Y | $R_1$ | $R_2$ | boiling point | liquid condition | IR-absorption spectrum |
|---|---|---|---|---|---|---|---|---|
| 1 | 2-OMe 5-OMe | 2 | $-CH_2CH_2-$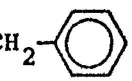 | $-CH_3$ | $-CH_2CH=C(CH_3)_2$ | * | pale yellow oil | νCO:1640 $cm^{-1}$ |
| 2 | 4-OMe | 1 | $-CH_2CH_2-$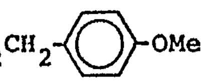 | $-CH_3$ | $-CH_2CH=C(CH_3)_2$ | * | pale yellow oil | νCO:1635 $cm^{-1}$ |
| 3 | 3-OMe 4-OMe | 2 | $-CH_2CH_2-$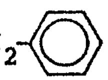 | $-CH_3$ | $-CH_2CH=C(CH_3)_2$ | * | pale yellow oil | νCO:1640 $cm^{-1}$ |
| 4 | | 0 | $-CH_2CH_2-$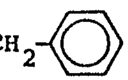 | $-CH_3$ | $-CH_2CH=C(CH_3)_2$ | 213-216°C (0.85 mmHg) | pale yellow oil | νCO:1635 $cm^{-1}$ |
| 5 | 4-OMe | 1 | $-CH_2CH_2-$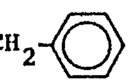 | $-CH_3$ | $-CH_2CH=C(CH_3)_2$ | 220-230°C (1 mmHg) | pale yellow oil | νCO:1640 $cm^{-1}$ |
| 6 | | 0 | $-CH_2CH_2-$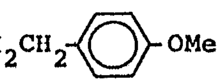 | $-CH_3$ | $-CH_2-$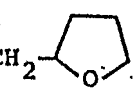 | * | pale yellow oil | νCO:1640 $cm^{-1}$ |

| Com. No. | X | n | Y | $R_1$ | $R_2$ | boiling point | liquid condition | IR-absorption spectrum |
|---|---|---|---|---|---|---|---|---|
| 7 | 2-OMe 5-OMe | 2 | $-CH_2CH_2-$ 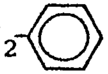 | $-CH_3$ | $-CH_2-$ 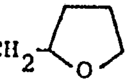 | * | pale yellow oil | νCO:1640 $cm^{-1}$ |
| 8 | 3-OMe | 1 | $-CH_2CH_2-$ 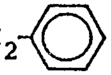 | $-CH_3$ | $-CH_2-$ 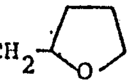 | * | pale yellow oil | νCO:1635 $cm^{-1}$ |
| 9 | 2-OMe | 1 | $-CH_2CH_2-$ 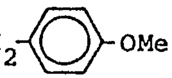 | $-CH_3$ | $-CH_2-$ 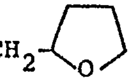 | * | pale yellow oil | νCO:1635 $cm^{-1}$ |
| 10 | | 0 | $-CH_2CH_2-$ 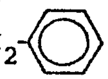 | $-CH_3$ | $-CH_2-$ 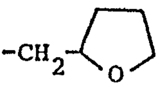 | * | yellow oil | νCO:1635 $cm^{-1}$ |
| 11 | 4-n-OPr | 1 | $-CH_2CH_2-$ 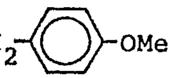 | $-CH_3$ | $-CH_2-$ 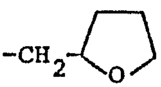 | * | yellow oil | νCO:1635 $cm^{-1}$ |
| 12 | | 0 | $-CH_2CH_2-$ 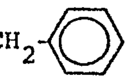 | $-n-C_3H_7$ | $-CH_2-$ 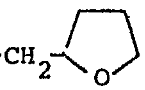 | * | yellow oil | νCO:1640 $cm^{-1}$ |
| 13 | 4-OMe | 1 | $-CH_2CH_2-$ 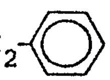 | $-n-C_3H_7$ | $-CH_2-$ 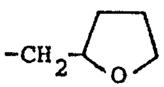 | * | yellow oil | νCO:1635 $cm^{-1}$ |
| 14 | 4-OMe | 1 | $-CH_2CH_2-$ 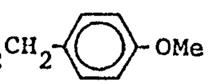 | $-CH_3$ | $-CH_2-$ 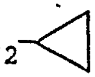 | * | pale yellow oil | νCO:1635 $cm^{-1}$ |

| Com. No. | X | n | Y | $R_1$ | $R_2$ | boiling point | liquid condition | IR-absorption spectrum |
|---|---|---|---|---|---|---|---|---|
| 15 | 4-OMe | 1 | $-CH_2CH_2-$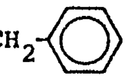 | $-CH_3$ | $-CH_2-$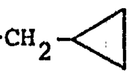 | 212-223°C (0.5 mmHg) | pale yellow oil | νCO:1640 $cm^{-1}$ |
| 16 | | 0 | $-CH_2CH_2-$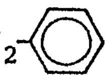 | $-CH_3$ | $-CH_2-$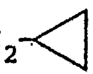 | * | pale yellow oil | νCO:1640 $cm^{-1}$ |
| 17 | 3-OMe<br>4-OMe | 2 | $-CH_2CH_2-$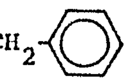 | $-CH_3$ | $-CH_2-$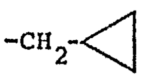 | * | pale yellow oil | νCO:1640 $cm^{-1}$ |
| 18 | 4-OMe | 1 | $-CH_2CH_2-$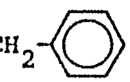 | $-CH_3$ | 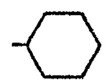 | 248-250°C (0.3 mmHg) | pale yellow oil | νCO:1640 $cm^{-1}$ |
| 19 | | 0 | $-CH_2CH_2-$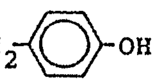-OH | $-CH_3$ | $-CH_2-$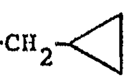 | * | pale yellow oil | νOH:3250 $cm^{-1}$<br>νCO:1620 $cm^{-1}$ |
| 20 | 2-OMe<br>5-OMe | 2 | $-CH_2CH_2-$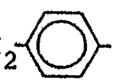-OH | $-CH_3$ | $-CH_2-$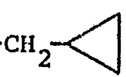 | * | pale yellow oil | νOH:3250 $cm^{-1}$<br>νCO:1620 $cm^{-1}$ |
| 21 | 2-OMe | 1 | $-CH_2CH_2-$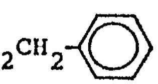 | $-CH_3$ | $-CH_2-$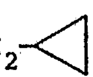 | * | yellow oil | νCO:1635 $cm^{-1}$ |
| 22 | 4-n-OPr | 1 | $-CH_2CH_2-$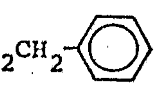 | $-CH_3$ | $-CH_2-$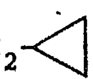 | * | pale yellow oil | νCO:1635 $cm^{-1}$ |

| Com. No. | X | n | Y | $R_1$ | $R_2$ | boiling point | liquid condition | IR-absorption spectrum |
|---|---|---|---|---|---|---|---|---|
| 23 | 3-OMe | 1 | $-CH_2CH_2-$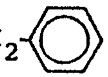 | $-CH_3$ | $-CH_2-$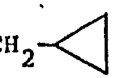 | * | pale yellow oil | $\nu CO$:1635 $cm^{-1}$ |
| 24 | 4-OMe | 1 | $-CH_2$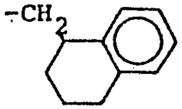 | $-CH_3$ | $-CH_2CH=C(CH_3)_2$ | * | pale yellow oil | $\nu CO$:1640 $cm^{-1}$ |
| 25 | 2-OMe 5-OMe | 2 | 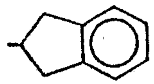 | $-CH_3$ | $-CH_2-$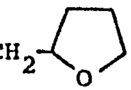 | * | pale yellow oil | $\nu CO$:1640 $cm^{-1}$ |
| 26 | 3-OMe 4-OMe | 2 | 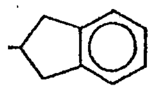 | $-CH_3$ | $-CH_2-$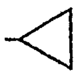 | * | pale yellow oil | $\nu CO$:1640 $cm^{-1}$ |
| 27 | 2-OMe | 1 | 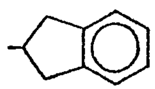 | $-CH_3$ | $-CH_2-$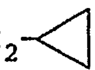 | * | pale yellow oil | $\nu CO$:1640 $cm^{-1}$ |
| 28 | | 0 | 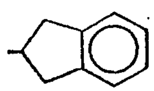 | $-CH_3$ | $-CH_2-$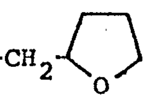 | * | pale yellow oil | $\nu CO$:1640 $cm^{-1}$ |
| 29 | 4-n-OPr | 1 | 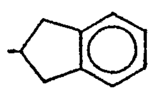 | $-CH_3$ | $-CH_2-$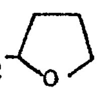 | * | pale yellow oil | $\nu CO$:1635 $cm^{-1}$ |

* The compounds were purified by column-chromatography using active alumina and a mixture of benzene and ethyl acetate as eluent.

EXAMPLE 9

In 30 ml of diethylether was dissolved 1 g of N-2-phenylethyl-[2-(N-methyl-N-allyl)aminoethyl]-4-methoxyphenylacetamide, and then an adequate amount of hydrogen chloride was bubbled into the solution. The mixture was concentrated to obtain 1 g of hydrochloric acid salt of N-2-phenylethyl-N-[2-(N-methyl-N-allyl)aminoethyl]-4-methoxyphenylacetamide as a colorless gelatin-like semi-solid substance having glassy appearance.

CLAIMS:

1. A phenylacetamide compound of the general formula:

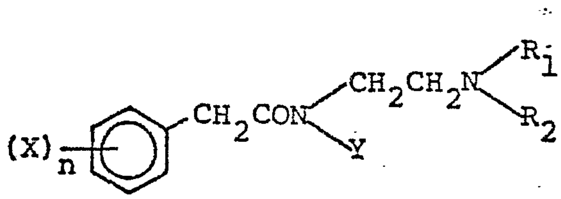

(I)

wherein $R_1$ is an alkyl group having 1 to 3 carbon atoms or a hydroxyalkyl group having 2 to 4 carbon atoms, $R_2$ is an alkenyl group having 3 to 6 carbon atoms, a cycloalkyl group having 4 to 7 carbon atoms, a cycloalkylalkyl group having 4 to 7 carbon atoms or tetrahydrofurfuryl group, X is an alkoxy group having 1 to 3 carbon atoms, n is an integer of 0 to 2, Y is 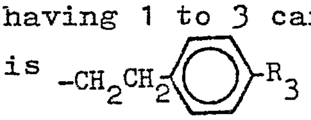 or 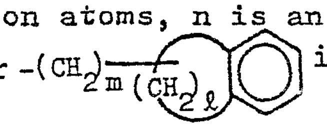 in which $R_3$ is a hydrogen atom, a hydroxyl group or an alkoxyl group having 1 to 3 carbon atoms, m is zero or 1, $\ell$ is an integer of 3 to 5; or a pharmaceutically acceptable salt thereof.

2. A compound as claimed in Claim 1, wherein n is zero.

3. A compound as claimed in Claim 1, wherein n is 1.

4. A compound as claimed in Claim 1, wherein n is 2.

5. A compound as claimed in Claim 3, wherein X is a methoxy group and is in the 4-position of the benzene ring.

6. A compound as claimed in Claim 5, wherein $R_1$ is a methyl group, $R_2$ is an allyl group and Y is a 2-phenylethyl group.

7. A compound as claimed in Claim 5, wherein $R_1$ is a methyl group, $R_2$ is a cyclopropylmethyl group and Y is a 2-phenylethyl group.

8. A compound as claimed in Claim 5, wherein $R_1$ is a methyl group, $R_2$ is a tetrahydrofurfuryl group and Y is a 2-phenylethyl group.

9. An analgesic composition comprising a therapeutically effective amount of at least one compound as claimed in Claim 1 and one or more pharmaceutically acceptable carriers or diluents.

10. For use in alleviating pain, a therapeutically effective amount of at least one compound as claimed in Claim 1.

AUSTRIAN CLAIMS:

1. A process for producing a phenylacetamide compound of the general formula:-

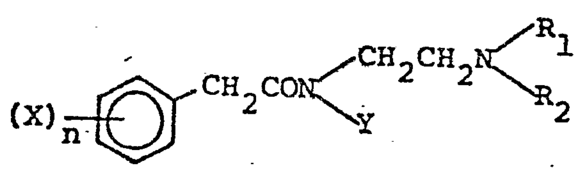

(I)

wherein $R_1$ is an alkyl group having 1 to 3 carbon atoms or a hydroxyalkyl group having 2 to 4 carbon atoms, $R_2$ is an alkenyl group having 3 to 6 carbon atoms, a cycloalkyl group having 4 to 7 carbon atoms, a cycloalkylalkyl group having 4 to 7 carbon atoms or tetrahydrofurfuryl group, X is an alkoxyl group having 1 to 3 carbon atoms, n is an integer of 0 to 2, Y is 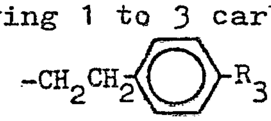 or 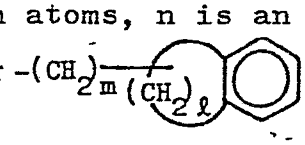

in which $R_3$ is a hydrogen atom, a hydroxyl group or an alkoxyl group having 1 to 3 carbon atoms, m is zero or 1, $\ell$ is an integer of 3 to 5; or a pharmaceutically acceptable salt thereof, comprising the step of reacting a phenylacetic acid of the general formula (II)

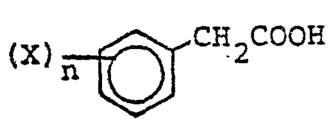

(II)

wherein X and n have the meaning given above, or a reactive functional derivative thereof with a compound of general

formula (III):

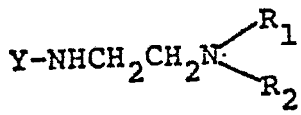

(III)

wherein Y, $R_1$ and $R_2$ have the meaning given above.

2. A process as claimed in Claim 1 wherein Y in the compound of formula (III) is 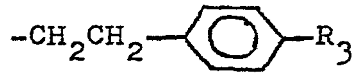 and $R_3$ is a hydroxyl group protected by a protective group and the method includes the further step of removing the protective group from the reaction product of the compounds of formulae (II) and (III).

3. A process for producing a phenylacetamide compound of the general formula:

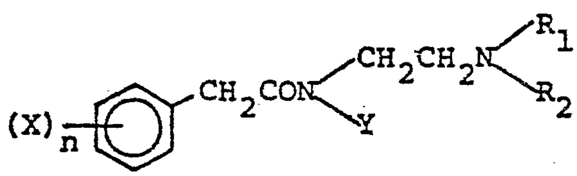

(I)

wherein $R_1$ is an alkyl group having 1 to 3 carbon atoms or a hydroxyalkyl group having 2 to 4 carbon atoms, $R_2$ is an alkenyl group having 3 to 6 carbon atoms, a cycloalkyl group having 4 to 7 carbon atoms, a cycloalkylalkyl group having 4 to 7 carbon atoms or tetrahydrofurfuryl group, X is an alkoxyl group having 1 to 3 carbon atoms, n is an integer of 0 to 2, Y is 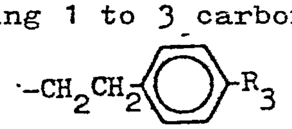 or 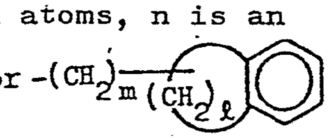

in which $R_3$ is a hydrogen atom, a hydroxyl group or an alkoxyl group having 1 to 3 carbon atoms, m is zero or 1, $\ell$ is an integer of 3 to 5; or a pharmaceutically acceptable salt thereof, using known starting materials and one or more process steps known per se.

4. A process as claimed in any preceding Claim, wherein n is zero.

5. A process as claimed in any one of Claims 1 to 3, wherein n is 1.

6. A process as claimed in any one of Claims 1 to 3, wherein n is 2.

7. A process as claimed in Claim 5, wherein X is a methoxy group and is in the 4-position of the benzene ring.

8. A process as claimed in Claim 7, wherein $R_1$ is a methyl group, $R_2$ is an allyl group and Y is a 2-phenylethyl group.

9. A process as claimed in Claim 7, wherein $R_1$ is a methyl group, $R_2$ is a cyclopropylmethyl group and Y is a 2-phenylethyl group.

10. A process as claimed in Claim 7, wherein $R_1$ is a methyl group, $R_2$ is a tetrahydrofurfuryl group and Y is a 2-phenylethyl group.

11. A method of alleviating pain using a therapeutically effective amount of a phenylacetamide compound of the general formula:-

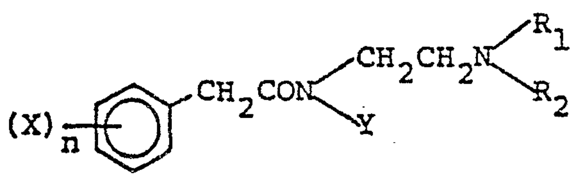

(I)

wherein $R_1$ is an alkyl group having 1 to 3 carbon atoms or a hydroxyalkyl group having 2 to 4 carbon atoms, $R_2$ is an alkenyl group having 3 to 6 carbon atoms, a cycloalkyl group having 4 to 7 carbon atoms, a cycloalkylalkyl group having 4 to 7 carbon atoms or tetrahydrofurfuryl group,

X is an alkoxy group having 1 to 3 carbon atoms, n is an integer of 0 to 2, Y is 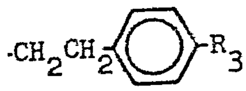 or 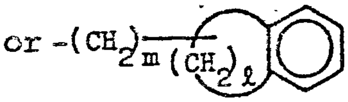

in which $R_3$ is a hydrogen atom, a hydroxyl group or an alkoxyl group having 1 to 3 carbon atoms, m is zero or 1, ℓ is an integer of 3 to 5; or a pharmaceutically acceptable salt thereof.

0017376

Application number

European Patent Office

# EUROPEAN SEARCH REPORT

EP 80 30 0817

## DOCUMENTS CONSIDERED TO BE RELEVANT

| Category | Citation of document with indication, where appropriate, of relevant passages | Relevant to claim |
|---|---|---|
| | US - A - 2 670 371 (J.W. CUSIC) * Claims; column 2, lines 7-10 * | 1,9,10 |
| | -- | |
| | GB - A - 964 712 (OLIN MATHIESON) * Page 1; page 2, lines 24-35 * | 1,9,10 |
| | -- | |
| | BE - A - 870 992 (KISSEI PHARMACEUTICAL) * Claims * & GB - A - 2 006 196 | 1,9,10 |
| | -- | |
| D | JOURNAL OF PHARMACEUTICAL SCIENCES, vol. 55, no. 10, October 1966, J.F. STUBBINS et al.: "Synthetic analgenics I", pages 1093-1096 * Page 1093, table I * | 1,9,10 |
| | ---- | |

CLASSIFICATION OF THE APPLICATION (Int. Cl. 3)

C 07 C 103/82
C 07 D 307/14
A 61 K 31/165
31/34

TECHNICAL FIELDS SEARCHED (Int.Cl. 3)

C 07 C 103/82
C 07 D 307/14
A 61 K 31/165
31/34

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 02-07-1980 | MOREAU |